Europäisches Patentamt

European Patent Office

Office européen des brevets

(11) Numéro de publication : **0 539 485 B1**

# (12) FASCICULE DE BREVET EUROPEEN

(45) Date de publication du fascicule du brevet :
**05.10.94 Bulletin 94/40**

(51) Int. Cl.⁵ : **G02B 7/00, F16M 11/12**

(21) Numéro de dépôt : **91913609.3**

(22) Date de dépôt : **15.07.91**

(86) Numéro de dépôt international :
**PCT/FR91/00583**

(87) Numéro de publication internationale :
**WO 92/01963 06.02.92 Gazette 92/04**

(54) DISPOSITIF DE SUPPORT ET DE POSITIONNEMENT D'UN MICROSCOPE.

(30) Priorité : **18.07.90 FR 9009159**

(43) Date de publication de la demande :
**05.05.93 Bulletin 93/18**

(45) Mention de la délivrance du brevet :
**05.10.94 Bulletin 94/40**

(84) Etats contractants désignés :
**AT BE CH DE DK ES FR GB GR IT LI LU NL SE**

(56) Documents cités :
**EP-A- 0 202 399**
**FR-A- 2 593 931**
**US-A- 4 531 816**

(73) Titulaire : **DEEMED International**
**2,avenue de Vignate**
**F-38610 Gière (FR)**

(72) Inventeur : **DRUAIS, Hervé**
**70, avenue du Vercors**
**F-38170 Seyssinet (FR)**

(74) Mandataire : **Kopacz, William James**
**BREESE-MAJEROWICZ,**
**CNIT-WTC1,**
**B.P. 434**
**F-92053 Paris La Défense (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un dispositif de support et de positionnement d'un microscope. Un tel dispositif trouve son application dans le domaine de la micro-chirurgie, ainsi que dans de nombreux autres domaines nécessitant un travail de grande précision sous assistance microscopique. On connait dans l'état de l'art des dispositifs motorisés de réglage de microscopes chirurgicaux dans lesquels l'appareil suspendu verticalement à un support ou sur un pied peut être déplacé sous 6 ou 7 degrés de liberté. Toutefois, lorsque la partie de l'objet observée n'est pas plane, ou lorsque la surface considérée est observée sous une certaine incidence, le déplacement entraîne une perte de la mise au point car les trajectoires générées ne se font pas dans l'axe optique. Il est alors nécessaire de revoir constamment la mise au point de l'objectif du microscope. Il en résulte un inconfort d'utilisation évident et fortement préjudiciable lors d'interventions micro-chirurgicales.

On a également proposé dans l'art antérieur des dispositifs dans lesquels le microscope est supporté de manière à pouvoir pivoter autour d'un premier axe perpendiculaire à l'axe optique, et selon un deuxième axe sensiblement vertical. Lorsque le grossissement du microscope est très faible, ce mode de réalisation est acceptable. Mais dans tous les cas une rotation se traduit pour l'observateur par un déplacement du point visé dans le champ optique. L'utilisateur est donc contraint de revoir continuellement le centrage du champ de visée. En outre, la mise au point n'est maintenue que pour certains types de déplacement.

Les possibilités de micro-manipulations ayant considérablement progressé tant dans le domaine de la chirurgie que dans d'autres domaines comme la mécanique de précision, il est apparu que les supports de microscope de type connu présentent des insuffisances handicapant l'opérateur. Celui-ci est contraint de consacrer une partie de son attention et de ses efforts au réglage du positionnement et de la mise au point du microscope.

Afin de remédier à ces inconvénients, la présente invention a pour objet la réalisation d'un support de microscope d'une grande stabilité et d'une conception ergonomique facilitant le positionnement du microscope par l'opérateur. De plus, le dispositif selon un mode de réalisation préféré permet de commander les déplacements du microscope selon des trajectoires asservies et contrôlées dans le référentiel comprenant l'axe de visée. La présente invention concerne plus particulièrement un dispositif de support et de positionnement d'un microscope, comportant un support apte à être lié à un référentiel fixe, et une platine apte à supporter un microscope, un élément mobile intermédiaire étant relié d'une part audit support par une première série d'organes articulés motorisés à structure parallèle assurant les trois degrés de liberté cartésiens et à ladite platine par une deuxième série d'organes motorisés à structure rotoïde de type poignet assurant 3 degrés de liberté en rotation. Selon un mode de réalisation particulier, le dispositif comporte en outre des moyens de commande générant des signaux de commande exploités par un calculateur pour assurer le déplacement du microscope dans le référentiel comprenant l'axe de visée du dit microscope, dans le plan perpendiculaire à l'axe de visée et/ou sur une sphère centrée sur le point focal-image de l'objectif du microscope et d'un rayon correspondant sensiblement à la distance focale du dit objectif.

L'opérateur est ainsi déchargé de toute intervention en ce qui concerne le contrôle de la mise au point ou de la trajectoire du microscope.

Selon un mode de réalisation préféré, la série d'organes articulés motorisés à structure parallèle assurant 3 degrés de liberté cartésiens est constituée par trois bras mobiles en rotation autour d'axes solidaires du dit support, lesdits axes étant coplanaires. L'autre extrémité de chacun desdits bras est reliée à l'élément mobile par au moins une barre de liaison. Trois actionneurs solidaires du support commandent les mouvements desdits bras mobiles. L'autre série d'organes articulés motorisés présente avantageusement trois degrés de liberté en rotation selon trois axes perpendiculaires. Ce qui fait 6 degrés de liberté soit 3 degrés de liberté en translation et 3 degrés de liberté en rotation.

Ce mode de réalisation permet la commande des trois degrés de liberté de base en parallèle à partir d'actionneurs supportés par la partie fixe du dispositif. Il en résulte une diminution de l'inertie et des contraintes s'exerçant sur les parties mobiles. Par ailleurs, de telles structures parallèles présentent une grande rigidité. Cette situation est bien entendu très avantageuse car elle permet d'améliorer la stabilité et la précision du dispositif.

Selon un mode de réalisation préféré, le dispositif comporte une poignée de commande droite et une poignée de commande gauche solidaires de ladite platine, comportant chacune trois capteurs de déplacement, un quatrième capteur de déplacement mesurant le déplacement des deux poignées par rapport à la platine. Les signaux générés par les capteurs sont traités pour générer des signaux $M_{di}$, $M_{gi}$ et $M_y$ représentant respectivement les déplacements de la poignée droite, de la poignée gauche et le déplacement rectiligne des deux poignées, avec i variant entre 1 et 3 désignant les trois directions des repères de référence des mesures. Les signaux sont convertis en informations de vitesse de translation V et de vitesse de rotation W selon les relations (en notant D la distance entre les poignées)

$$V_{px} = (M_{d1} + M_{g1})/2 \qquad W_{px} = (M_{g2} - M_{d2})/2/D$$
$$V_{py} = M_y \qquad W_{py} = (M_{d3} + M_{g3})/2$$
$$V_{pz} = (M_{d2} + M_{g2})/2 \qquad W_{pz} = (M_{d1} - M_{g1})/2/D$$

Ces vitesses sont exprimées dans un repère parallèle au repère des poignées, le centre de ce repère étant situé sur l'axe de visée. Ces informations de vitesse sont exploitées pour commander les mouvements du microscope.

Le dispositif ainsi réalisé présente une grande ergonomie et une facilité d'utilisation optimale. En particulier, les mouvements requis pour commander les poignées correspondent sensiblement aux mouvements qu'aurait l'opérateur pour déplacer un microscope non-asservi. En particulier, les poignées de commande étant disposées sur la platine porte-microscope, l'opérateur ressent physiquement l'effet de ces actions sur les poignées de commande. Il est ainsi intégré dans la chaîne.

Avantageusement, chacune des poignées de commande comporte des capteurs potentiométriques, l'un desdits capteurs mesurant la rotation de la poignée autour de l'axe passant par les deux poignées. Les signaux électriques provenant de ces capteurs sont convertis en signaux numériques par un convertisseur analogique-digital.

Avantageusement, les moyens de commande comportent un détecteur générant un signal de désactivation du frein lorsque l'opérateur agit sur lesdits moyens de commande. Le microscope est ainsi automatiquement stabilisé dès que l'opérateur cesse d'agir sur les commandes, il passe par une phase où les 6 axes sont asservis en position puis freinés.

Avantageusement, les signaux de vitesse de translation et de rotation sont filtrés pour limiter les variations à une valeur-seuil. Les déplacements intempestifs, pouvant dans certains cas présenter des dangers, sont ainsi évités. De tels mouvements pourraient se produire en cas d'action mal contrôlée de l'opérateur sur les moyens de commande, en cas d'ordre de commandes incompatibles avec les limites de résistance mécanique du dispositif, ou encore en cas de heurt accidentel des poignées de commande par une personne ou un objet.

Selon un mode de réalisation particulier, le dispositif selon la présente invention comporte un mode de déplacements lent rectiligne du microscope dans lequel le microscope se déplace dans un repère lié aux poignées en mode cartésien, en fonction des actions exercées sur l'une au moins des poignées de commande. Il s'agit d'un système à pantin intégré. L'opérateur peut ainsi contrôler des déplacements de très faible amplitude avec une seule main, pendant qu'une main au moins est libre pour le travail de précision en cours. De même, avec une pédale de commande il peut générer un déplacement dans le repère lié à l'axe de visée, les deux mains étant libres.

Selon une autre variante, le dispositif comporte un mode de déplacement lent sphérique du microscope dans lequel le microscope se déplace sur une sphère centrée sur le point visé, en fonction des actions exercées sur l'une au moins des poignées de commande. Ce déplacement sphérique peut être commandé soit à partir des poignées de commande, soit à partir d'une pédale complémentaire. Le paramètre correspondant à la distance focale définissant le rayon de la sphère de déplacement est enregistré dans le calculateur lors de la configuration du système dans le fichier paramètre.

La présente invention sera descrite plus en détail dans ce qui suit, faisant référence aux dessins où :
- la figure 1 représente une vue schématique du dispositif,
- la figure 2 représente une vue schématique des moyens de commande,
- la figure 3 représente une vue détaillée de la première série d'organes articulés.

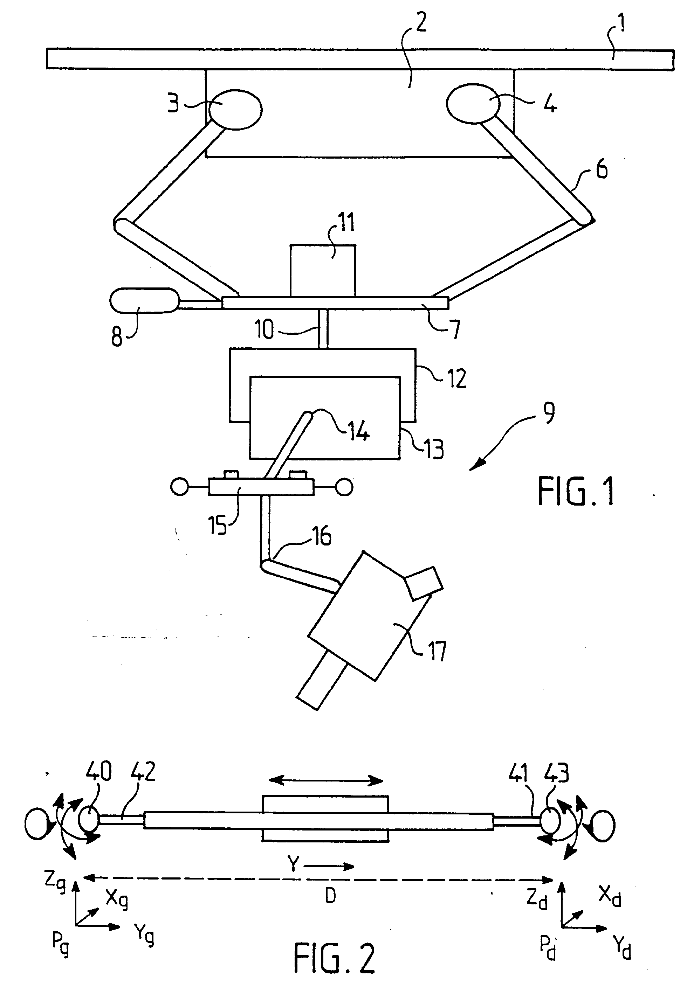

La figure 1 représente une vue simplifiée d'un exemple de réalisation du dispositif selon l'invention.

Il est fixé au plafond, par exemple d'un bloc opératoire de micro-chirurgie, par l'intermédiaire d'un rail de prépositionnement (1). Ce rail de prépositionnement permet de positionner l'ensemble du dispositif au-dessus du champ opératoire. Ce rail de prépositionnement (1) guide un support (2) comportant trois moteurs (3,4,5) commandant les mouvements d'une première série (6) d'organes articulés. Cette première série d'organes articulés est constituée par un système à trois axes "delta parallèle" qui sera décrite de façon plus détaillée en figure 2. Elle est reliée à un élément mobile intermédiaire (7). Cet élément intermédiaire (7) se déplace parallèlement au support (2), et donc dans la plupart des cas dans un plan horizontal. Il est muni d'une poignée de prépositionnement (8) permettant de déplacer l'ensemble du dispositif le long du rail de guidage (1). L'élément mobile intermédiaire (7) supporte la deuxième série d'organes articulés (9), comportant trois axes de pivotement orthogonaux. Le premier de ces axes est un axe vertical (10) commandé par un moteur (11) disposé sur l'élément mobile intermédiaire (7). Cet axe vertical (10) est solidaire d'un cadre (12) présentant un axe de rotation perpendiculaire (13) . Cet axe est relié à une pièce intermédiaire supportant deux moteurs commandant les mouvements selon les deux axes (13,14) perpendiculaires entre eux et à l'axe vertical (10). Les moyens de commande (15) sont solidaires de l'axe (14) d'ordre 6 perpendiculaire aux axes d'ordre 4 (13) et d'ordre 5 (10) mentionnés dans ce qui précède. Le microscope (17) est solidaire de cet axe (14) par le biais d'un axe de rotation supplémentaire d'ordre 7. Cet ordre de prépositionnement (16) permet d'ajuster la direction

EP 0 539 485 B1

de la visée selon le type d'opération envisagée.

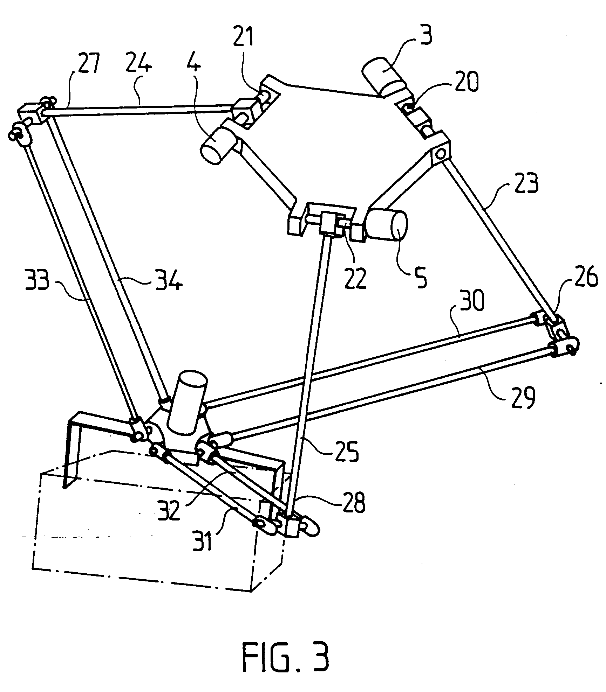

La figure 3 représente une vue détaillée de la réalisation de la première série d'organes articulés (6).

Le support (2) comporte trois moteurs (3,4,5) comportant chacun une partie fixe solidaire du support, et dont les axes respectivement (20,21,22) sont coplanaires. Des bras de commande (23,24,25) sont solidaires desdits axes respectivement (20,21,22) . Ces bras sont montés de façon rigide sur lesdits axes, leurs axes longitudinaux étant perpendiculaires à l'axe correspondant. L'autre extrémité (26 à 28) de chacun des bras (23 à 25) est solidaire de deux bras de liaison respectivement (29,30), (31,32) et (33,34) par deux doubles articulations formées de 2 axes rotoïdes perpendiculaires entre eux. Chacune des paires de bras de liaisons est reliée d'autre part par des doubles articulations formées de 2 axes rotoïdes perpendiculaires entre eux à l'élément mobile intermédiaire (7) . La disposition à 120° des bras articulés a été exposée à titre d'exemple préférentiel car cette géométrie offre une bonne rigidité. Il ne s'agit toutefois pas d'un mode de réalisation limitatif, et de nombreuses autres formes de réalisation sont envisageables.

La figure 2 représente un vue détaillée d'un mode de réalisation des moyens de commande, mettant en oeuvre une poignée gauche (40) et une poignée droite (41). Comme indiqué précédemment, les moyens de commande sont solidaires de l'axe (14) d'ordre 6, afin de transmettre à l'opérateur des impressions ergonomiques.

Les poignées sont du type "manche à balai" est agissent de façon différentielle. Elles comportent chacune un manche (42, 43) monté sur une rotule autorisant des débattements angulaires dans deux plans perpendiculaires. Ces débattements sont symbolisés sur la figure 3 par des flèches. Les manches peuvent également être manoeuvrés en rotation autour de leurs axes longitudinaux. En outre les deux poignées peuvent être déplacées simultanément en translation selon l'axe longitudinal.

Les déplacements des manches (42,43) sont transmis à des potentiomètres qui délivrent des signaux analogiques. Les valeurs potentiométriques sont converties en signaux numériques par un convertisseur analogique-digital 12 bits et traitées numériquement pour obtenir les vitesses de translation et de rotation. Bien entendu, on peut envisager d'utiliser d'autres types de moyens de commande, mettant en oeuvre différents capteurs connus dans l'état de l'art. Les vitesses sont déterminées par les relations suivantes:

$$V_{px} = (M_{d1} + M_{g1})/2 \qquad W_{px} = (M_{g2} - M_{d2})/2/D$$
$$V_{py} = M_y \qquad W_{py} = (M_{d3} + M_{g3})/2$$
$$V_{pz} = (M_{d2} + M_{g2})/2 \qquad W_{pz} = (M_{d1} - M_{g1})/2/D$$

avec

$M_{d1}$: déplacement du manche droit 43 selon l'axe $OX_d$

$M_{g1}$: déplacement du manche gauche selon l'axe $OX_g$

$M_{d2}$: déplacement du manche droit 43 selon l'axe $OZ_d$

$M_{g2}$: déplacement du manche gauche selon l'axe $OZ_g$

$M_{d3}$: rotation du manche droit 43 autour de l'axe $O_{Yd}$

$M_{g3}$ : rotation du manche gauche autour de l'axe $OY_g$

$M_y$: déplacement en translation des moyens de commande selon l'axe longitudinal Y.

Les données numériques sont traitées de façon à écrêter les valeurs supérieures à un seuil réglable et les variations supérieures à un autre seuil réglable, afin d'éviter les déplacements trop brusques dépassant les capacités de résistance mécanique du dispositif, du microscope, ou de l'utilisateur.

Le traitement du signal se fait de façon non linéaire sur la norme de vitesse afin de permettre de contrôler aussi bien un déplacement précis à vitesse très lente qu'un déplacement à des vitesses élevées.

Les actionneurs des organes articulés sont commandés par un calculateur réalisant les opérations suivantes:
- acquisition des signaux analogiques de commande générés par les poignées de contrôle,
- conversion analogique-digital de ces signaux,

4

- filtrage des signaux numériques pour éliminer les données intempestives et délivrer des signaux numériques correspondant aux vitesses de translation et de rotation désirées,
- acquisition des signaux correspondant à la position du système à un instant T et transfert par le modèle géométrique direct pour obtenir les positions dans l'espace cartésien,
- transfert par le modèle cinématique inverse à partir des positions cartésiennes courantes et des vitesses cartésiennes de translation et de rotation désirées pour obtenir les consignes de mouvement du système de l'instant T,
- filtrage pour éliminer les données intempestives,
- gestion des lois de mouvements et des asservissements,
- conversion digitale-analogique des consignes de mouvement,
- gestion des commandes ergonomiques du sytème.

Ces signaux contrôlent les mouvements des actionneurs des organes articulés.

Le calculateur a par ailleurs d'autres fonctions telles que la gestion des sécurités, et le calcul de la position et du déplacement du microscope dans le référentiel fixe, ou dans un référentiel déterminé par l'opérateur.

Ce calculateur est selon l'exemple décrit constitué par un micro-ordinateur de type connu relié à des circuits électroniques d'acquisition de signaux et à une carte spécialisée de commande d'axes (CAID) produit de DMD, à un panneau de contrôle et à un étage de puissance délivrant les signaux de consigne.

Le calculateur gère trois modes de commandes. Le premier mode "axes freinés" est le mode par défaut en l'absence d'action sur les organes de commande.

Le second mode "déplacement rapide" permet à l'opérateur de commander les déplacements du microscope à des vitesses proportionnelles aux déplacements appliqués sur les poignées et sur le capteur de déplacement rectiligne selon l'axe longitudinal. Il est commandé par les poignées agissant de façon différentielle pour les commandes de rotation et de translation. La vitesse maximale autorisée est réglable par un potentiomètre disposé sur le panneau de contrôle. Ce paramètre est laissé en partie à l'appréciation de l'opérateur. Ce mode de commande n'est possible que tant que l'opérateur agit physiquement sur les poignées de contrôle, faute de quoi le dispositif revient automatiquement en mode "axes freinés". La détection de l'action de l'opérateur s'effectue par des capteurs intégrés dans les poignées de contrôle.

Le troisième mode "déplacement lent" est un mode permettant de déplacer le robot suivant les directions XYZ d'un référentiel cartésien lié à l'axe de visée du microscope. Il est commandé soit par la poignée droite, soit par la poignée gauche, soit par une pédale accessoire.

Le calculateur exploite les signaux provenant des poignées de contrôle ou de la pédale pour délivrer des signaux de consigne correspondant à des trajectoires planes perpendiculaires à l'axe de visée ou des trajectoires sphériques autour du point de visée, selon une sphère dont le rayon correspond à la distance de mise au point et des translations suivant l'axe de visée, en fonction des actions sur les poignées de contrôle ou sur la pédale. Les deux modes rectilignes et sphériques sont complémentaires.

La présente invention a été décrite dans ce qui précède à titre d'exemple non limitatif. Il est bien évident que l'homme du métier sera à même de réaliser de nombreuses variantes sans pour autant sortir du cadre de l'invention.

**Revendications**

1. Dispositif de support et de positionnement d'un microscope, comportant un support (2) apte à être lié à un référentiel fixe, et une platine apte à supporter un microscope (17), un élément mobile intermédiaire (7) étant relié d'une part audit support (2) par une première série d'organes (6) articulés motorisés dont l'architecture de type parallèle assure trois degrés de libertés cartésiens, et à ladite platine par une deuxième série d'organes motorisés (9), le dispositif comportant des moyens de commande générant des signaux de commande exploités par un calculateur pour asservir le déplacement du microscope.

2. Dispositif selon la revendication 1, caractérisé en ce que la première série d'organes (6) articulés motorisés est constituée par trois bras mobiles (23, 24, 25) en rotation autour d'axes (20, 21, 22) solidaires dudit support (2), lesdits axes (20, 21, 22) étant coplanaires, l'autre extrémité de chacun desdits bras (23, 24, 25) étant reliée à l'élément mobile (7) par au moins une barre de liaison, trois actionneurs solidaires (3, 4, 5) du support (2) commandant les mouvements desdits bras mobiles.

3. Dispositif selon la revendication 1 ou 2, caractérisé en ce que les moyens de commande sont constitués d'une poignée droite (41) et d'une poignée gauche (40) solidaires par l'intermédiaire de 4 capteurs de déplacement de l'organe (14), de ladite platine, comportant chacune trois capteurs de déplacement, un quatrième capteur de déplacement mesurant le déplacement des deux poignées (40, 41) par rapport à la platine, les signaux générés par les capteurs étant traités pour générer des signaux $M_{di}$, $M_{gi}$ et $M_y$ représentant respectivement les déplacements de la poignée droite (41), de la poignée gauche (40) et le déplacement rectiligne des deux poignées (40, 41), avec i variant entre 1 et 3 désignant les trois directions des repères de référence des mesures, lesdits signaux étant convertis en informations de vitesse de translation V et de vitesse de rotation W selon les relations

$$V_{px} = (M_{d1} + M_{g1})/2 \qquad W_{px} = (M_{g2} - M_{d2})/2/D$$
$$V_{py} = M_y \qquad W_{py} = (M_{d3} + M_{g3})/2$$
$$V_{pz} = (M_{d2} + M_{g2})/2 \qquad W_{pz} = (M_{d1} - M_{g1})/2/D$$

ces vitesses étant exprimées dans un repère parallèle au repère des poignées, le centre de ce repère étant situé sur l'axe de visée, ces informations de vitesse étant exploitées pour commander les mouvements du microscope.

4. Dispositif selon la revendication 3, caractérisé en ce que chacune des poignées de commande (40, 41) comporte des capteurs potentiométriques, l'un desdits capteurs mesurant la rotation de la poignée autour de l'axe passant par les deux poignées (40, 41).

5. Dispositif selon l'une quelconque des revendications 1 à 4, caractérisé en ce que les moyens de commande comportent un détecteur générant un signal de désactivation de la position freinée lorsque l'opérateur agit sur-lesdits moyens de commande.

6. Dispositif selon l'une quelconque des revendications 1 à 5 caractérisé en ce que les signaux de vitesse de translation et de rotation sont filtrés pour limiter les variations à une valeur-seuil.

7. Dispositif selon l'une quelconque des revendications 1 à 6, caractérisé en ce qu'il comporte un mode de déplacement lent rectiligne du microscope dans lequel le microscope se déplace dans un référentiel lié aux repères des poignées, en fonction des actions exercées sur l'une des poignées de commande ou sur une pédale.

8. Dispositif selon l'une quelconque des revendications 1 à 7, caractérisé en ce qu'il comporte un mode de déplacement lent sphérique du microscope dans lequel le microscope se déplace sur une sphère centrée sur le point visé, en fonction des actions exercées sur l'une au moins des poignées de commande ou sur une pédale de commande.

9. Dispositif selon l'une quelconque des revendications 1 à 8, caractérisé en ce qu'il comporte une commande d'arrêt d'urgence assurant le repliement rapide des moyens articulés.


**Patentansprüche**

1. Halte- und Positioniervorrichtung für ein Mikroskop, umfassend eine an einem ortsfesten Bezugspunkt befestigbare Halterung (2) und eine Grundplatte, welche ein Mikroskop (17) tragen kann, wobei ein bewegliches Zwischenglied (7) einerseits mittels einer ersten Reihe von motorisierten Gelenkorganen (6), deren parallele Anordnung drei Freiheitsgrade nach dem cartesischen Koordinatensystem sicherstellt, mit der Halterung (2) und andererseits mittels einer zweiten Reihe von motorisierten Organen (9) mit der Grundplatte verbunden ist, und wobei die Vorrichtung Steuermittel aufweist, welche von einem Rechner zu verarbeitende Steuersignale erzeugen, um das Verstellen des Mikroskops zu regeln.

2. Vorrichtung nach Anspruch 1,
dadurch gekennzeichnet, daß die erste Reihe von motorisierten Gelenkorganen (6) aus drei beweglichen Armen (23, 24, 25) besteht, welche um mit der Halterung (2) zusammenwirkende Achsen (20, 21, 22) rotieren, wobei die genanten Achsen (20, 21, 22) in der selben Ebene liegen, das andere Ende eines jeden Armes (23, 24, 25) mit dem beweglichen Glied (7) mittels mindestens einer Verbindungsstange verbunden ist und drei mit der Halterung (2) zusammenwirkende Betätigungsglieder (3, 4, 5) die Bewegungen der beweglichen Arme steuern.

3. Vorrichtung gemäß Anspruch 1 oder 2,
dadurch gekennzeichnet, daß die Steuermittel aus einem rechten (41) und einem linken Griff (40) bestehen, welche über vier Wegaufnehmer für das Organ (14) mit der Grundplatte zusammenwirken, wobei jeder Griff drei Wegaufnehmer aufweist und ein vierter Wegaufnehmer den Weg der beiden Handgriffe (40, 41) im Verhältnis zur Grundplatte mißt, und wobei die von den Aufnehmern erzeugten Signale verarbeitet werden zur Erzeugung von Signalen $M_{di}$, $M_{gi}$ und $M_y$, welche jeweils den Weg des rechten Griffs (41), des linken Griffs (40) und den geradlinigen Weg der beiden Griffe (40, 41) angeben, wobei i zwischen 1 und 3 variiert und die drei Richtungen der Bezugspunkte der Meßreferenz darstellen soll, und wobei die Signale in Informationen für die Translationsgeschwindigkeit V und für die Rotationsgeschwindigkeit W nach den folgenden Relationen übertragen werden:

$$V_{px} = (M_{d1} + M_{g1})/2 \qquad W_{px} = (M_{g2} - M_{d2})/2/D$$
$$V_{py} = M_y \qquad W_{py} = (M_{d3} + M_{g3})/2$$
$$V_{pz} = (M_{d2} + M_{g2})/2 \qquad W_{pz} = (M_{d1} - M_{g1})/2/D,$$

wobei diese Geschwindigkeitswerte in einem zum Bezugspunkt der Handgriffe parallelen Bezugspunkt ausgedrückt sind, dessen Zentrum auf der Sucherachse liegt, und die Geschwindigkeitsinformationen genutzt werden, um die Bewegungen des Mikroskops zu steuern.

4.  Vorrichtung nach Anspruch 3,
    dadurch gekennzeichnet, daß jeder der Steuergriffe (40, 41) Potentiometer umfaßt, von denen eines die Rotation des Griffes um die durch beide Handgriffe (40, 41) durchgehende Achse mißt.

5.  Vorrichtung nach einem der Ansprüche 1 bis 4,
    dadurch gekennzeichnet, daß die Steuermittel einen Fühler umfassen, der ein Desaktivierungssignal der Bremsposition erzeugt, wenn der Benutzer die Steuermittel betätigt.

6.  Vorrichtung nach einem der Ansprüche 1 bis 5,
    dadurch gekennzeichnet, daß die Signale für die Translations- und die Rotationsgeschwindigkeit gefiltert werden, um die Variationen auf einen Schwellenwert zu begrenzen.

7.  Vorrichtung nach einem der Ansprüche 1 bis 6,
    dadurch gekennzeichnet, daß sie einen langsamen, geradlinigen Verstellmodus des Mikroskops aufweist, in welchem das Mikroskop sich in einem mit den Bezugspunkten der Griffe verbundenen Bezugssystem bewegt, abhängig von der Betätigung der Steuergriffe oder eines Fußpedals.

8.  Vorrichtung nach einem der Ansprüche 1 bis 7,
    dadurch gekennzeichnet, daß sie einen langsamen, sphärenförmigen Bewegungsmodus des Mikroskops aufweist, in welchem das Mikroskop sich auf einer auf den anvisierten Punkt zentrierten Sphäre bewegt, abhängig von der Betätigung wenigstens eines der Steuergriffe oder eines Steuerpedals.

9.  Vorrichtung nach einem der Ansprüche 1 bis 8,
    dadurch gekennzeichnet, daß sie eine Notunterbrechungssteuerung aufweist, um den schnellen Rückzug der Gelenkglieder zu ermöglichen.

## Claims

1.  A device for carrying and positioning a microscope, including a bracket (2) adapted to be referred to a fixed datum frame, and a platen adapted to support a microscope (17), with an intermediate movable element (7) being connected, on one hand, to said bracket (2) through a first set of powered hinge members (6), the parallel type architecture of which ensures three cartesian degrees of freedom and on the other hand, to said platen through a second set of powered members (9), said device comprising control means with produce control signals for use by a computer in order to command said microscope movement.

2.  The device as recited in claim 1, characterized in that said first set of powered hinge members (6) includes three movable arms (23, 24, 25) rotating about axles (20, 21, 22) integral said bracket (2), said axles (20, 21, 22) being coplanar, the other end of each of said arms (23, 24, 25) being connected to said movable element (7) by means of at least a connecting bar; and with three actuators (3, 4, 5) integral with said bracket (2) controlling said movable arm movements.

3.  The device as recited in claim 1 or 2, characterized in that said control means include a right-hand handle (41) and a left-hand handle (40) integral with said platen member (14) through four movement sensors, each including three movement sensors, a fourth movement sensor measuring both handles movement (40, 41) relative to said platen, the signals produced by said sensors being processed in order to generate signals $M_{di}$, $M_{gi}$ and $M_y$ which respectively represent movements of right-hand handle (41), left-hand handle (40) and rectilinear movement of both handles (40, 41), with i varying between 1 and 3 and designating the three directions of measuring reference marks, said signals being converted into translation speed V data and rotation speed W data according to relations as follows :

$$V_{px} = (M_{d1} + M_{g1})/2 \qquad W_{px} = (M_{g2} - M_{d2})/2/D$$
$$V_{py} = M_y \qquad W_{py} = (M_{d3} + M_{g3})/2$$
$$V_{pz} = (M_{d2} + M_{g2})/2 \qquad W_{pz} = (M_{d1} - M_{g1})/2/D$$

said speeds being expressed by a mark in parallel relationship with handles' mark, said mark center being located in the sight axle, and said speed data being used to command microscope movements.

4. The device as recited in claim 3, characterized in that each of said control handles (40, 41) includes potentiometric sensors, with one of said sensors measuring the handle rotation about an axle passing through both handles (40, 41).

5. The device as recited in any one of claims 1-4, characterized in that said control means include a detector which produces a signal for desactivating the braked position when operator is acting on said control means.

6. The device as recited in any one of claims 1-5, characterized in that translation and rotation speed signals are filtered with a view to limiting variations thereof to a threshold value.

7. The device as recited in any one of claims 1-6, characterized in that said device includes a rectilinear slow displacement mode for said microscope wherein said microscope moves in a datum frame in relation with handles marks, depending on operations which are carried out on one of said control handles or on a pedal.

8. The device as recited in any one of claims 1-7, characterized in that said device includes a spherical slow displacement mode for said microscope wherein said microscope moves upon a sphere centered on the aimed point, depending on operations which are carried out on at least one of said control handles or on a control pedal.

9. The device as recited in any one of claims 1-8, characterized in that said device includes an emergency stop control, thereby a fast folding of said hinge means is obtained.

FIG.1

FIG.2

9

FIG. 3